(19) Europäisches Patentamt
European Patent Office
Office européen des brevets

(11) EP 0 463 407 B1

(12) **EUROPEAN PATENT SPECIFICATION**

(45) Date of publication and mention of the grant of the patent:
**13.02.2002 Bulletin 2002/07**

(51) Int Cl.[7]: **A61K 31/444**, A61K 31/496, A61K 31/4427, A61K 31/4422, A61P 9/04

(21) Application number: **91109081.9**

(22) Date of filing: **04.06.1991**

(54) **Use of dihydropyridins in cardiotonic pharmaceutical compositions**

Verwendung von Dihydropyridinen in kardiotonischen pharmazeutischen Zusammensetzungen

Utilisation des dérivés de la dihydropyridine dans des compositions pharmaceutiques cardiotoniques

(84) Designated Contracting States:
**BE CH DE DK ES FR GB IT LI NL SE**

(30) Priority: **25.06.1990 JP 16609390**

(43) Date of publication of application:
**02.01.1992 Bulletin 1992/01**

(73) Proprietor: **Mitsubishi Pharma Corporation**
**Osaka-shi, Osaka 541-0046 (JP)**

(72) Inventors:
- **Uchida, Takeshi, c/o The Green Cross Corporation Hirakata-shi, Osaka 573 (JP)**
- **Ohtaki, Yutaka, c/o The Green Cross Corporation Hirakata-shi, Osaka 573 (JP)**
- **Kido, Hideaki, c/o The Green Cross Corporation Hirakata-shi, Osaka 573 (JP)**
- **Watanabe, Masahiro, c/o The Green Cross Corp. Hirakata-shi, Osaka 573 (JP)**

(74) Representative:
**von Kreisler, Alek, Dipl.-Chem. et al**
**Patentanwälte von Kreisler-Selting-Werner**
**Postfach 10 22 41**
**50462 Köln (DE)**

(56) References cited:

| | |
|---|---|
| **EP-A- 0 257 616** | **EP-A- 0 342 577** |
| **EP-A- 0 379 737** | **US-A- 4 735 956** |

- **FOLIA PHARMACOLOGICA JAPONICA, vol. 95, no. 4, April 1990, pages 177-184; H. KIDO et al.: "Angiographic studies on the effects of a novel calcium antagonist AE0047 on cerebral arteries in dogs, with special reference to the preventive effects of AE0047 on cerebral vasoconstriction induced by endothelin"**
- **IDEM**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).



Printed by Jouve, 75001 PARIS (FR)

## Description

The present invention relates to the use of a dihydropyridine derivative having a particular structure mentioned below for the production of a cardiotonic medicament for increasing cardiac output and maximal differential value in left ventricle pulse.

Cardiotonic medicaments are drugs which reinforce myocardial contractive force, thereby improving cardiac functions. Included among them are those act directly on cardiac muscle to enhance contractive force and those stimulate cardiac functions indirectly by acting on peripheral vessels (resistance vessel, capacitance vessel) or central nerve system.

Heretofore, cardiac glucosides (digitalis preparations) have been used as a cardiotonic drug. It is not known, however, that dihydropyridine derivatives have mild and long-lasting cardiotonic action (e.g., 4-heteroaryl-1,4-dihydropyridines are known from U.S. Patent No. 4,735,956, out of which some may be useful as cardiotonic agents).

The cardiotonic agents so far known are generally quick-acting and do not last for a long time. Pharmaceuticals having mild and long-lasting cardiotonic action are advantageous in that they are easily administered to patients with chronic heart failure such as cardiac insufficiency, and development of such cardiotonic drugs is demanded.

SUMMARY OF THE INVENTION

An object of the invention is to provide cardiotonic medicament having mild and long-lasting cardiotonic action.

In an attempt to achieve the aforementioned object, the present inventors have conducted intensive studies and found that a dihydropyridine derivative having a particular structure mentioned below has a cardiotonic action, specifically mild and long-lasting cardiotonic action, which resulted in completion of the invention.

DETAILED DESCRIPTION OF THE INVENTION

The present invention provides the use of a dihydropyridine derivative of the formula (I) [hereinafter referred to as dihydropyridine derivative (I)]

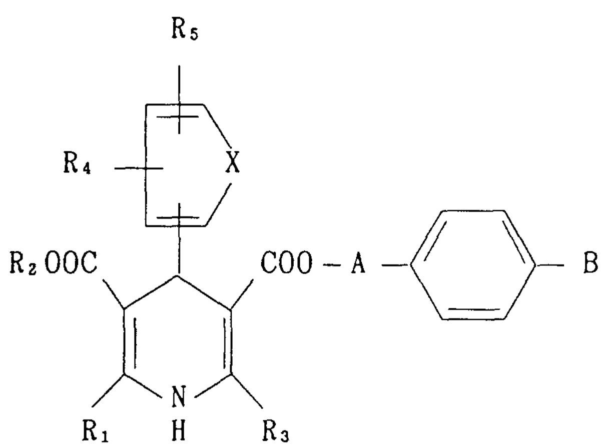

(I)

wherein:

$R_1$, $R_2$ and $R_3$ are the same or different and are an alkyl having 1 to 6 carbon atoms which may be substituted by cycloalkyl having 3 to 6 carbon atoms, a cycloalkyl having 3 to 6 carbon atoms or an alkoxyalkyl having 3 to 7 carbon atoms;
$R_4$ and $R_5$ are the same or different and are hydrogen, halogen, nitro, a halogenated alkyl having 1 to 6 carbon atoms, an alkylsulfonyl having 1 to 6 carbon atoms, a halogenated alkoxy having 1 to 6 carbon atoms, an alkylsulfinyl having 1 to 6 carbon atoms, an alkyl having 1 to 6 carbon atoms which may be substituted by cycloalkyl having 3 to 6 carbon atoms, a cycloalkyl having 3 to 6 carbon atoms, an alkoxy having 1 to 3 carbon atoms, cyano, an alkoxycarbonyl having 2 to 4 carbon atoms or an alkylthio having 1 to 3 carbon atoms, where $R_4$ and $R_5$ are not hydrogen at the same time;
X is vinylene or azomethine;
A is an alkylene having 2 to 4 carbon atoms; and

B is $-N(R_6)_2$ or

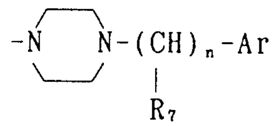

where

$R_6$ and $R_7$ are independently hydrogen, an alkyl having 1 to 6 carbon atoms which may be substituted by cycloalkyl having 3 to 6 carbon atoms, a cycloalkyl having 3 to 6 carbon atoms, an aralkyl, an aryl or pyridyl,
Ar is an aryl or pyridyl and
n is an integer of 0 to 2;

or an acid addition salt thereof and a pharmaceutically acceptable carrier or excipient, for the production of a cardiotonic medicament for increasing cardiac output and maximum differential value in left ventricle pulse.

The dihydropyridine derivatives (I) and acid addition salts thereof used in the present invention are specifically characterized in their mild action, long-lasting effect and low toxicity, which contribute to their efficacy and safety.

In formula (I), the alkyl having 1 to 6 carbon atoms represented by $R_1$, $R_2$ or $R_3$ may have a straight- or branched-chain and is examplified by methyl, ethyl, propyl, isopropyl, butyl, isobutyl, sec-butyl, t-butyl, pentyl, isopentyl, neopentyl and hexyl, with preference given to those having 1 to 4 carbon atoms. The alkyl may have a cycloalkyl having 3 to 6 carbon atoms on the alkyl terminal, such as cyclopropylmethyl, cyclobutylethyl and cyclopentylmethyl.

The cycloalkyl having 3 to 6 carbon atoms represented by $R_1$, $R_2$ or $R_3$ is exemplified by cyclopropyl, cyclobutyl, cyclopentyl and cyclohexyl.

The alkoxyalkyl having 3 to 7 carbon atoms represented by $R_1$, $R_2$ or $R_3$ is exemplified by methoxyethyl, ethoxyethyl, propoxyethyl, isopropoxyethyl, butoxyethyl, methoxypropyl, 2-methoxy-1-methylethyl and 2-ethoxy-1-methylethyl.

The substituent represented by $R_4$ or $R_5$ may be the same or different, and may bind to any position of the ring, with preference given to the 2- and/or 3-position to the binding site with the dihydropyridine ring. As the halogen at $R_4$ or $R_5$, exemplified are fluorine, chlorine, bromine and iodine, and particularly preferred are fluorine atom and chlorine atom, and as the alkyl and the cycloalkyl, preferred are those mentioned as $R_1$ to $R_3$. The alkoxy having 1 to 3 carbon atoms and the alkylthio having 1 to 3 carbon atoms are exemplified by methoxy, ethoxy, propoxy and isopropoxy, and methylthio, ethylthio, propylthio and isopropylthio, respectively. As the alkoxycarbonyl having 2 to 4 Carbon atoms there may be mentioned methoxycarbonyl and ethoxycarbonyl. Halogen of halogenated substituents is exemplified by those mentioned above, and the halogenated alkyl may be that wherein some of the hydrogen atoms are replaced with halogen atoms [e.g. $(CF_3)_2CHCH_2$-, $CF_3CH_2$-] or all of the hydrogen atoms are replaced with halogen atoms, such as trifluoromethyl, Also, the halogenated alkoxy may be that wherein some of the hydrogen atoms are replaced with halogen atoms or all of the hydrogen atoms are replaced with halogen atoms. The halogenated alkyl and halogenated alkoxy have preferably 1 to 4 carbon atoms. Examples of the alkyl moiety in the alkylsulfonyl and in the alkylsulfinyl include those exemplified as $R_1$ to $R_3$, namely those having 1 to 6, preferably those having 1 to 4 carbon atoms. As $R_4$ and $R_5$, preferred are cyano and halogenated alkyl (particularly, trifluoromethyl).

The alkyl and the cycloalkyl represented by $R_6$ or $R_7$ include those exemplified as $R_1$ to $R_3$. As the aralkyl, preferred are phenyl $C_{1-3}$ alkyl such as benzyl, $\alpha$-phenylethyl, $\beta$-phenylethyl and $\gamma$-phenylpropyl. As the aryl, mention may be made of phenyl and naphthyl. These aromatic rings may have the same or different substituents at optional positions. The substituents on the aromatic ring include those mentioned as $R_4$ and $R_5$. The pyridyl includes 2-pyridyl, 3-pyridyl and 4-pyridyl, which may have the substituents mentioned above as $R_4$ and $R_5$.

The alkylene having 2 to 4 carbon atoms represented by A may have a straight- or branched-chain and is exemplified by ethylene, trimethylene, tetramethylene and 1,2-dimethylethylene.

The aryl and the pyridyl represented by Ar include those exemplified as $R_6$ and $R_7$ and may have the same substituents.

The ring represented by

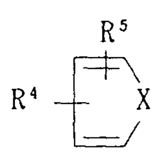

which is the 4-position substituent of dihydropyridine, means a benzene ring when X is vinylene (-CH=CH-) and pyridine when X is azomethine (-CH=N-). At an optional position, the ring may bind to the 4-position of the dihydropyridine.

The substituents $R_4$ and $R_5$ may be at any position of ortho-, meta- and para-positions to a carbon atom binding to the 4-position of the dihydropyridine, with preference given to the ortho- and/or meta-position.

As the dihydropyridine derivetives (I) and their acid addition salts, there may be mentioned, for example, 2-[p-(4-benzhydrylpiperazino)phenyl]ethyl methyl 2,6-dimethyl-4-(3-nitrophenyl)-1,4-dihydropyridine-3,5-dicarboxylate, 2-[p-(4-benzhydrylpiperazino)phenyl]ethyl methyl 2,6-dimethyl-4-(4-cyano-2-pyridyl)-1,4-dihydropyridine-3,5-dicarboxylate and their acid addition salts.

The dihydropyridine derivatives (I) can be produced by reacting an optional portion constituting the dihydropyridine derivatives (I) and the residual part by a method known per se, particularly by cyclodehydration.

Specifically, the dihydropyridine derivatives (I) can be produced by the methods described in USPs 4849429, 4910195, 4886819 and 4892875, and Japanese Patent Unexamined Publication No. 260064/1986.

The dihydropyridine derivatives (I) produced as described can be obtained at an optional purity by known separation and purification means such as concentration, extraction, chromatography, reprecipitation and recrystallization.

Since the dihydropyridine derivatives (I) have a basic group, they can be converted to acid addition salts by a known means. As such salts, no limitation is posed thereon as long as they are pharmacologically acceptable and nontoxic and examples thereof include salts with inorganic acids such as hydrochloride, hydrobromide, phosphate and sulfate, and salts with organic acids such as acetate, succinate, malate, fumarate, maleate and tartrate.

The dihydropyridine derivatives (I) and acid addition salts thereof which are the active ingredient of the medicament of the invention are extremely low toxic and exhibit mild and long-lasting cardiotonic action (cardiac function improving action) on mammals such as mouse, rat, rabbit, dog, cat and human.

Thus, the medicament is useful for the treatment and prevention of heart failure, specifically chronic heart failure.

The medicament of the invention lessens heart load by way of decreased blood pressure and improves cardiac functions by increased blood stream by way of cardiovasodilation action.

When the dihydropyridine derivatives (I) or acid addition salts thereof are used to prepare the medicament mentioned above they can be mixed with pharmaceutically required ingredients such as pharmacologically acceptable additives such as carrier, excipient and diluent to give pharmaceutical compositions in a form of powders, granules, tablets, capsules and injections, which can be orally or parenterally administered. The dihydropyridine derivatives (I) or their acid addition salts are incorporated in the above-mentioned pharmaceutical compositions in an effective amount. While the dosage varies depending on the administration route, gravity of diseases, body weight or age of patients, they are preferably administered in an amount of 0.05 to 20 mg/kg body weight/day, preferably 0.1 to 4 mg/kg body weight/day in one to several divided doses a day when administering orally to an adult.

In the case of the intraveneous administration, they are preferably administered in an amount of 0.1 to 300 μg/kg body weight/day, preferably 5 to 100 μg/kg body weight/day in one to several divided doses a day.

The present invention is hereinbelow described in more detail by illustrating experiment examples, working examples and reference examples.

As regards $^1$H-NMR, used was $CDCl_3$ unless otherwise specified.

Experiment Example 1 (toxicity)

$LD_{50}$ was estimated using male mice of 10-11 weeks old weighing 14-16 g (3 to 5 mice per group). All the $LD_{50}$ values obtained were at least >1100 mg/kg body weight and those of most compounds were >1400 mg/kg body weight.

Thus, the dihydropyridine derivatives (I) of the invention show significantly high acute toxicity value in comparison with known compounds and are safer.

Experiment Example 2

Using male and female mongrel dogs weighing 7 to 12 kg (3 to 4 dogs per one group, Keari Co., Ltd.), effects of intraveneously administered dihydropyridine derivatives (I) or their acid addition salts on circulartory kinetics and cardiac functions were examined. Namely, mean central venous pressure (MCVP), maximum and minimum differential values in left ventricle pulse (Max dp/dt, Min dp/dt) and cardiac output (CO) were measured.
Test drug : Compound 2 to be mentioned below which is an acid addition salt of the dihydropyridine derivatives (I) was dissolved in ethanol and Tween® 80, and diluted with physiological saline where the final concentration of Tween® 80 was not more than 0.03%. The compound 2 was bolus administered into the right vein at a dose of 30 μg/kg in a 1 ml/kg volume. As the control, 0.03% Tween® 80 physiological saline was administered at a 1 ml/kg volume.

Method : A dog anesthesized with sodium pentobarbital (25 mg/kg) was fixed at the dorsal position.

Mean central venous pressure (MCVP) was measured by a piezoelectric transducer (Stetham P-50; Gould Corp.) via a polyethylene catheter inserted in the right femoral vein.

Maximum and minimum differential values in left ventricle pulse (Max dp/dt, Min dp/dt) were measured using a microtip-transducer (Nippon Koden) inserted in the left ventricle through the left carotid artery.

Cardiac output (CO) was measured by a thermodilution cardiac output measurement apparatus (MLC-4100, Nippon Koden) via a Swan-Ganz catheter inserted in pulmonary artery from the left jugular vein.

The results are summarized in Tables 1 to 4 in mean values.

Table 1 Cardiac Output (CO)

| | Immediately before | 1 min | 5 min | 10 min | 30 min | 60 min | 120 min |
|---|---|---|---|---|---|---|---|
| Control | 100 | 102 | 99 | 103 | 98 | 100 | 93 |
| Compound 2 | 100 | 114 | 125 | 140 | 142 | 149 | 147 |

Table 2 Mean Central Vein Pressure (MCVP)

| | Immediately before | 1 min | 3 min | 5 min | 10 min | 30 min | 60 min | 120 min |
|---|---|---|---|---|---|---|---|---|
| Control | 100 | 97 | 102 | 98 | 98 | 97 | 102 | 103 |
| Compound 2 | 100 | 95 | 96 | 98 | 106 | 99 | 103 | 93 |

Table 3 Maximum Differential Values in Left Ventricle Pulse (Max dp/dt)

| | Immediately before | 1 min | 3 min | 5 min | 10 min | 30 min | 60 min | 120 min |
|---|---|---|---|---|---|---|---|---|
| Control | 100 | 103 | 103 | 102 | 103 | 102 | 104 | 105 |
| Compound 2 | 100 | 102 | 105 | 105 | 107 | 120 | 125 | 127 |

Table 4 Minimum Differential Values in Left Ventricle Pulse (Min dp/dt)

| | Immediately before | 1 min | 3 min | 5 min | 10 min | 30 min | 60 min | 120 min |
|---|---|---|---|---|---|---|---|---|
| Control | 100 | 104 | 105 | 104 | 105 | 110 | 111 | 112 |
| Compound 2 | 100 | 98 | 101 | 107 | 102 | 87 | 83 | 85 |

From the results in Tables, it is evident that the dihydropyridine derivatives (I) show moderate expression of cardiotonic action and have markedly long durability of the action. In addition, increase in cardiac output (CO) and maximum differential value in left ventricle pulse (Max dp/dt) confirmed the cardiotonic action. The dihydropyridine derivatives (I) show reducing of post-load caused by hypotensive action and increase of coronary blood flow at the same time, which indicates less actual stress on the heart. Furthermore, decrease of mean central venous pressure proves their efficacy as a cardiotonic agent.

Reference Example 1

Synthesis of 2-[p-(4-benzhydrylpiperazino)phenyl]ethyl methyl 2,6-dimethyl-4-(3-nitrophenyl)-1,4-dihydropyridine-3,5-dicarboxylate (Compound 1) and hydrochloride thereof (Compound 2) :

In a 100 ml-eggplant type flask were charged 3-nitrobenzaldehyde (1.144 g, 7.57 mmol), [p-(4-benzhydrylpiperazino)-phenyl]ethyl acetoacetate (3.464 g, 7.59 mmol) and methyl 3-aminocrotonate (873 mg, 7.58 mmol). Thereto was added isopropanol (12 ml). The flask was equipped with a Dimroth condenser, and subjected to 16 hours' reflux under heating. The reaction solvent was distilled off under reduced pressure and the residue was separated by column chromatography [silica gel,chloroform - methanol (45:1)] and column chromatography [silica gel,ethyl acetate - n-hexane (2:3)], and the crude product obtained was purified by high performance liquid chromatography to give 2.503 g of the title Compound 1 (yield 48%).
IR $\nu_{max}^{KBr}$ $cm^{-1}$ : 1680, 1520
$^1$H-NMRδ :
8.06 (1H, t, J=2Hz), 7.97 (1H, ddd, J=8; 2; 1Hz), 7.1-7.6 (12H), 7.03 (2H, d, J=8.6Hz), 6.80 (2H, d, J=8.6Hz), 6.02 (1H, s), 5.07 (1H, s), 4.26 (1H, s), 4.22 (2H, t, J=7Hz), 3.64 (3H, s), 3.15 (4H, dd, J=5; 4.7Hz), 2.81 (2H, t, J=7Hz), 2.55 (4H, dd, J=5; 4.7Hz), 2.33, 2.28 (3H, s, respectively)

The Compound 1 (2.124 g, 3.16 mmol) was placed in a 200 ml-eggplant type flask and the flask was rubber-sealed. Methylene chloride (100 ml) was added thereto, and after dissolution of the content, the mixture was stirred at room temperature for 30 minutes while introducing hydrogen chloride gas. The resultant crystals were filtered off to give about 2.22 g of the title Compound 2.
IR $\nu_{max}^{KBr}$ $cm^{-1}$ : 2450, 1680, 1525, 1350
$^1$H-NMR δ :
13.72 (1H, brs), 8.05-7.9 (6H), 7.82, 7.26 (4H, $A_2B_2$·q, J=8.6Hz), 7.6-7.3 (8H), 6.28 (1H, s), 5.2-5.05 (2H), 5.01 (2H, s), 4.27 (2H, t, J=6.5Hz), 4.3-4.1 (2H), 3.66 (3H, s), 3.65-3.45 (4H), 2.95 (2H, t, J=6.5Hz), 2.36, 2.33 (3H, s, respectively)

Example 1

| Tablets : | | |
|---|---|---|
| (1) | Compound 2 | 10 g |
| (2) | Fine granule No. 209 for direct compression (Fuji Kagakusha) | 110 g |
| | Magnesium metasilicate alminate | 20% |
| | Corn starch | 30% |
| | Lactose | 50% |
| (3) | Crystalline cellulose | 60 g |
| (4) | CMC calcium | 18 g |
| (5) | Magnesium stearate | 2 g |

(1), (3) and (4) were passed through a 100 mesh-sieve in advance. (1), (3), (4) and (2) were respectively dried to a certain water content, after which the mixture was kneaded at the above weight ratio by a mixing machine. (5) was added to the homogeneously-mixed powder and was mixed for a short time (30 seconds). The mixed powder was compressed into tablets of 200 mg each.

The tablets may be gastro-coated using a film coating agent such as polyvinyl acetal diethylaminoacetate or coated with a food colouring.

Example 2

| Capsules : | |
|---|---|
| (1) Compound 2 | 50 g |
| (2) Lactose | 930 g |

(continued)

| Capsules : | |
|---|---|
| (3) Magnesium stearate | 20 g |

The above ingredients were weighed and homogeneously mixed, after which the mixed powder was charged in hard gelatin capsules at 200 mg each.

Example 3

| Injections : | |
|---|---|
| (1) Compound 2 | 5 mg |
| (2) Glucose | 100 mg |
| (3) Physiological saline | 10 ml |

The mixed solution of the above was filtered through a membrane filter, after which it was sterilized by filtration. The filtrate was aseptically poured into a vial, charged with nitrogen gas and sealed to afford an intraveneous injection.

## Claims

### Claims for the following Contracting States : BE, CH, LI, DE, DK, FR, GB, IT, NL, SE

1. Use of a dihydropyridine derivative of the formula (I)

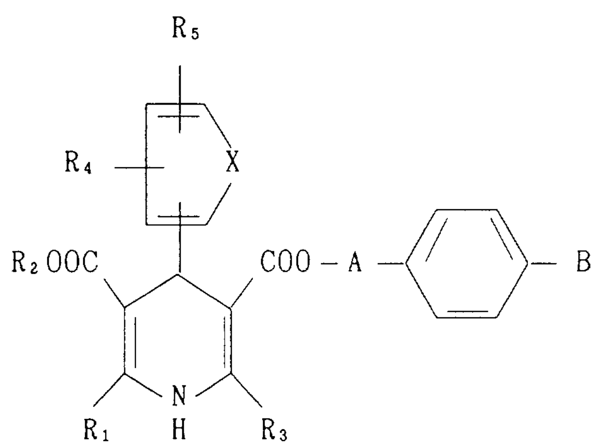

(I)

wherein:

$R_1$, $R_2$ and $R_3$ are the same or different and are an alkyl having 1 to 6 carbon atoms which may be substituted by cycloalkyl having 3 to 6 carbon atoms, a cycloalkyl having 3 to 6 carbon atoms or an alkoxyalkyl having 3 to 7 carbon atoms;
$R_4$ and $R_5$ are the same or different and are hydrogen, halogen, nitro, a halogenated alkyl having 1 to 6 carbon atoms, an alkylsulfonyl having 1 to 6 carbon atoms, a halogenated alkoxy having 1 to 6 carbon atoms, an alkylsulfinyl having 1 to 6 carbon atoms, an alkyl having 1 to 6 carbon atoms which may be substituted by cycloalkyl having 3 to 6 carbon atoms, a cycloalkyl having 3 to 6 carbon atoms, an alkoxy having 1 to 3 carbon atoms, cyano, an alkoxycarbonyl having 2 to 4 carbon atoms or an alkylthio having 1 to 3 carbon atoms, where $R_4$ and $R_5$ are not hydrogen at the same time;
X is vinylene or azomethine;

A is an alkylene having 2 to 4 carbon atoms; and
B is $-N(R_6)_2$ or

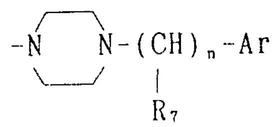

where
$R_6$ and $R_7$ are independently hydrogen, an alkyl having 1 to 6 carbon atoms which may be substituted by cycloalkyl having 3 to 6 carbon atoms, a cycloalkyl having 3 to 6 carbon atoms, an aralkyl, an aryl or pyridyl,
Ar is an aryl or pyridyl and
n is an integer of 0 to 2;

or an acid addition salt thereof and a pharmaceutically acceptable carrier or excipient, for the production of a cardiotonic medicament for increasing cardiac output and maximum differential value in left ventricle pulse.

**2.** Use as claimed in claim 1, whereby in the dihydropyridine derivative of the formula (I)

$R_1$, $R_2$ and $R_3$ are the same or different and are an alkyl;
$R_4$ is hydrogen;
$R_5$ is nitro, a halogenated alkyl or cyano;
$R_6$ is an alkyl, an aralkyl or an aryl;
$R_7$ is an aryl;
Ar is an aryl; and
n is 1.

**3.** Use as claimed in claim 1, wherein the dihydropyridine derivative is selected from 2-[p-(4-benzhydrylpiperazino) phenyl]ethyl methyl 2,6-dimethyl-4-(3-nitrophenyl)-1,4-dihydropyridine-3,5-dicarboxylate and 2-[p-(4-benzhydrylpiperazino)phenyl]ethyl methyl 2,6-dimethyl-4-(4-cyano-2-pyridyl)-1,4-dihydropyridine-3,5-dicarboxylate and their acid addition salts.

**4.** Use as claimed in claim 1, wherein the dyhydropyridine derivative is selected from 2- [p-(4-benzhydrylpiperazino) phenyl]ethyl methyl 2,6-dimethyl-4-(3-nitrophenyl)-1,4-dihydropyridine-3,5-dicarboxylate and 2-[p-(4-benzhydrylpiperazino)phenyl]-ethyl methyl 2,6-dimethyl-4-(4-cyano-2-pyridyl)-1,4-dihydropyridine-3,5-dicarboxylate and hydrochlorides thereof.

**5.** Use as claimed in claim 1, wherein the medicament is suitable for the treatment of heart failure.

**6.** Use as claimed in claim 1 or 2, whereby in the dihydropyridine derivative of formula (I) X is vinylene.

**7.** Use as claimed in claim 6, wherein the dihydropyridine derivative is 2-[p-(4-benzhydrylpiperazino)phenyl]ethyl methyl 2,6-dimethyl-4-(3-nitrophenyl)-1,4-dihydropyridine-3,5-dicarboxylate or an acid addition salt thereof.

**8.** Use as claimed in claim 6, wherein the dihydropyridine derivative is 2-[p-(4-benzhydrylpiperazino)phenyl]ethyl methyl 2,6-dimethyl-4-(3-nitrophenyl)-1,4-dihydropyridine-3,5-dicarboxylate or a hydrochloride thereof.

**9.** Use as claimed in claim 6, wherein the medicament is suitable for the treatment of heart failure.

**Claims for the following Contracting State : ES**

**1.** A method to manufacture a cardiotonic medicament for increasing cardiac output and maximum differential value in left ventricle pulse **characterized in** the use of a dihydropyridine derivative of the formula

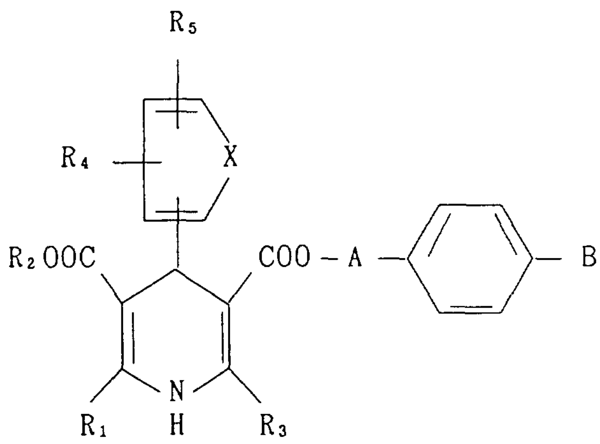

(I)

wherein:

$R_1$, $R_2$ and $R_3$ are the same or different and are an alkyl having 1 to 6 carbon atoms which may be substituted by cycloalkyl having 3 to 6 carbon atoms, a cycloalkyl having 3 to 6 carbon atoms or an alkoxyalkyl having 3 to 7 carbon atoms;
$R_4$ and $R_5$ are the same or different and are hydrogen halogen, nitro, a halogenated alkyl having 1 to 6 carbon atoms, an alkylsulfonyl having 1 to 6 carbon atoms, a halogenated alkoxy having 1 to 6 carbon atoms, an alkylsulfinyl having 1 to 6 carbon atoms, an alkyl having 1 to 6 carbon atoms which may be substituted by cycloalkyl having 3 to 6 carbon atoms, a cycloalkyl having 3 to 6 carbon atoms, an alkoxy having 1 to 3 carbon atoms, cyano, an alkoxycarbonyl having 2 to 4 carbon atoms or an alkylthio having 1 to 3 carbon atoms, where $R_4$ and $R_5$ are not hydrogen at the same time;
X is vinylene or azomethine,
A is an alkylene having 2 to 4 carbon atoms; and
B is $-N(R_6)_2$ or

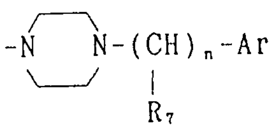

where
$R_6$ and $R_7$ are independently hydrogen, an alkyl having 1 to 6 carbon atoms which may be substituted by cycloalkyl having 3 to 6 carbon atoms, a cycloalkyl having 3 to 6 carbon atoms, an aralkyl, an aryl or pyridyl,
Ar is an aryl or pyridyl and
n is an integer of 0 to 2;

or an acid addition salt thereof and a pharmaceutically acceptable carrier or excipient.

2. The method as claimed in claim 1, whereby in the dihydropyridine derivative of the formula (I)

$R_1$, $R_2$ and $R_3$ are the same or different and are an alkyl;
$R_4$ is hydrogen;
$R_5$ is nitro, a halogenated alkyl or cyano;
$R_6$ is an alkyl, an aralkyl or an aryl;
$R_7$ is an aryl;
Ar is an aryl; and
n is 1.

3. The method as claimed in claim 1, wherein the dihydropyridine derivative is selected from 2-[p-(4-benzhydrylpiperazino)phenyl]ethylmethyl2,6-dimethyl-4-(3-nitrophenyl)-1,4-dihydropyridine-3,5-dicarboxylateand2-[p-(4-benzhydrylpiperazino)phenyl]ethyl methyl 2,6-dimethyl-4-(4-cyano-2-pyridyl)-1,4-dihydropyridine-3,5-dicarboxylate and

their acid addition salts.

**4.** The method as claimed in claim 1, wherein the dyhydropyridine derivative is selected from 2-[p-(4-benzhydrylpiperazino)phenyl]ethylmethyl2,6-dimethyl-4-(3-nitrophenyl)-1,4-dihydropyridine-3,5-dicarboxylateand2-[p-(4-benzhydrylpiperazino)phenyl]-ethyl methyl 2,6-dimethyl-4-(4-cyano-2-pyridyl)-1,4-dihydropyridine-3,5-dicarboxylate and hydrochlorides thereof.

**5.** The method as claimed in claim 1, wherein the medicament is suitable for the treatment of heart failure.

**6.** The method as claimed in claim 1 or 2, whereby in the dihydropyridine derivative of formula (I) X is vinylene.

**7.** The method as claimed in claim 6, wherein the dihydropyridine derivative is 2-[p-(4-benzhydrylpiperazino)phenyl] ethyl methyl 2,6-dimethyl-4-(3-nitrophenyl)-1,4-dihydropyridine-3,5-dicarboxylate or an acid addition salt thereof.

**8.** The method as claimed in claim 6, wherein the dihydropyridine derivative is 2-[p-(4-benzhydrylpiperazino)phenyl] ethyl methyl 2,6-dimethyl-4-(3-nitrophenyl)-1,4-dihydropyridine-3,5-dicarboxylate or a hydrochloride thereof.

**9.** The method as claimed in claim 6, wherein the medicament is suitable for the treatment of heart failure.

**Patentansprüche**

**Patentansprüche für folgende Vertragsstaaten : BE, CH, LI, DE, DK, FR, GB, IT, NL, SE**

**1.** Verwendung eines Dihydropyridin-Derivats der Formel (I)

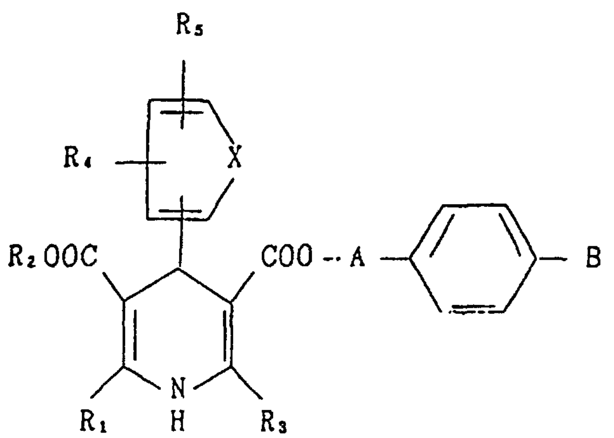

(I)

wobei:

$R_1$, $R_2$ und $R_3$ gleich oder verschieden sind und ein Alkyl mit 1 bis 6 Kohlenstoffatomen, das mit einem Cycloalkyl mit 3 bis 6 Kohlenstoffatomen substituiert sein kann, ein Cycloalkyl mit 3 bis 6 Kohlenstoffatomen oder ein Alkoxyalkyl mit 3 bis 7 Kohlenstoffatomen darstellen;

$R_4$ und $R_5$ gleich oder verschieden sind und Wasserstoff, Halogen, Nitro, ein halogeniertes Alkyl mit 1 bis 6 Kohlenstoffatomen, ein Alkylsulfonyl mit 1 bis 6 Kohlenstoffatomen, ein halogeniertes Alkoxy mit 1 bis 6 Kohlenstoffatomen, ein Alkylsulfinyl mit 1 bis 6 Kohlenstoffatomen, ein Alkyl mit 1 bis 6 Kohlenstoffatomen, das mit einem Cycloalkyl mit 3 bis 6 Kohlenstoffatomen substituiert sein kann, ein Cycloalkyl mit 3 bis 6 Kohlenstoffatomen, ein Alkoxy mit 1 bis 3 Kohlenstoffatomen, Cyan, ein Alkoxycarbonyl mit 2 bis 4 Kohlenstoffatomen oder ein Alkylthio mit 1 bis 3 Kohlenstoffatomen darstellen, wobei $R_4$ und $R_5$ nicht gleichzeitig Wasserstoff sind;

X Vinylen oder Azomethin ist;

A ein Alkylen mit 2 bis 4 Kohlenstoffatomen ist; und

B = $-N(R_6)_2$ oder

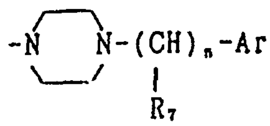

ist;
wobei

$R_6$ und $R_7$ unabhängig voneinander Wasserstoff, ein Alkyl mit 1 bis 6 Kohlenstoffatomen, das mit einem Cycloalkyl mit 3 bis 6 Kohlenstoffatomen substituiert sein kann, ein Cycloalkyl mit 3 bis 6 Kohlenstoffatomen, ein Aralkyl, ein Aryl oder Pyridyl sein kann;

Ar ein Aryl oder Pyridyl ist; und

n eine ganze Zahl von 0 bis 2 ist;

oder eines Säureadditionssalzes davon und eines pharmazeutisch annehmbaren Trägers oder Excipienten zur Herstellung eines kardiotonischen Medikaments zur Erhöhung der Herzförderleistung und des maximalen Differentialwerts im Linkskammerpuls.

**2.** Verwendung gemäß Anspruch 1, wobei im Dihydropyridin-Derivat der Formel (I)

$R_1$, $R_2$ und $R_3$ gleich oder verschieden sind und ein Alkyl darstellen;
$R_4$ Wasserstoff ist;
$R_5$ Nitro, ein halogeniertes Alkyl oder Cyan ist;
$R_6$ ein Alkyl, ein Aralkyl oder ein Aryl ist;
$R_7$ ein Aryl ist;
Ar ein Aryl ist; und
n = 1 ist.

**3.** Verwendung gemäß Anspruch 1, wobei das Dihydropyridin-Derivat aus 2-[p-(4-Benzhydrylpiperazino)phenyl]ethylmethyl-2,6-dimethyl-4-(3-nitrophenyl)-1,4-dihydropyridin-3,5-dicarboxylat und 2-[p-(4-Benzhydrylpiperazino)phenyl]ethylmethyl-2,6-dimethyl-4-(4-cyan-2-pyridyl)-1,4-dihydropyridin-3,5-dicarboxylat und ihren Säureadditionssalzen ausgewählt ist.

**4.** Verwendung gemäß Anspruch 1, wobei das Dihydropyridin-Derivat aus 2-[p-(4-Benzhydrylpiperazino)phenyl]ethylmethyl-2,6-dimethyl-4-(3-nitrophenyl)-1,4-dihydropyridin-3,5-dicarboxylat und 2-[p-(4-Benzhydryipiperazino)phenyl]ethylmethyl-2,6-dimethyl-4-(4-cyan-2-pyridyl)-1,4-dihydropyridin-3,5-dicarboxylat und ihren Hydrochloriden ausgewählt ist.

**5.** Verwendung gemäß Anspruch 1, wobei das Medikament für die Behandlung von Herzversagen geeignet ist.

**6.** Verwendung gemäß Anspruch 1 oder 2, wobei X im Dihydropyridin-Derivat der Formel (I) Vinylen ist.

**7.** Verwendung gemäß Anspruch 6, wobei es sich bei dem Dihydropyridin-Derivat um 2-[p-(4-Benzhydrylpiperazino)phenyl]ethylmethyl-2,6-dimethyl-4-(3-nitrophenyl)-1,4-dihydropyridin-3,5-dicarboxylat oder ein Säureadditionssalz davon handelt.

**8.** Verwendung gemäß Anspruch 6, wobei es sich bei dem Dihydropyridin-Derivat um 2-[p-(4-Benzhydrylpiperazino)phenyl]ethylmethyl-2,6-dimethyl-4-(3-nitrophenyl)-1,4-dihydropyridin-3,5-dicarboxylat oder ein Hydrochlorid davon handelt.

**9.** Verwendung gemäß Anspruch 6, wobei das Medikament für die Behandlung von Herzversagen geeignet ist.

**Patentansprüche für folgenden Vertragsstaat : ES**

1. Verfahren zur Herstellung eines kardiotonischen Medikaments zur Erhöhung der Herzförderleistung und des maximalen Differentialwerts im Linkskammerpuls, **gekennzeichnet durch** die Verwendung eines Dihydropyridin-Derivats der Formel

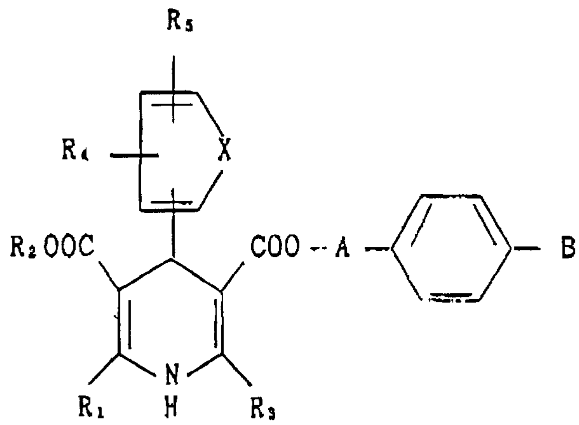

(I)

wobei:

$R_1$, $R_2$ und $R_3$ gleich oder verschieden sind und ein Atkyl mit 1 bis 6 Kohlenstoffatomen, das mit einem Cycloalkyl mit 3 bis 6 Kohlenstoffatomen substituiert sein kann, ein Cycloalkyl mit 3 bis 6 Kohlenstoffatomen oder ein Alkoxyalkyl mit 3 bis 7 Kohlenstoffatomen darstellen;

$R_4$ und $R_5$ gleich oder verschieden sind und Wasserstoff, Halogen, Nitro, ein halogeniertes Alkyl mit 1 bis 6 Kohlenstoffatomen, ein Alkylsulfonyl mit 1 bis 6 Kohlenstoffatomen, ein halogeniertes Alkoxy mit 1 bis 6 Kohlenstoffatomen, ein Alkylsulfinyl mit 1 bis 6 Kohlenstoffatomen, ein Alkyl mit 1 bis 6 Kohlenstoffatomen, das mit einem Cycloalkyl mit 3 bis 6 Kohlenstoffatomen substituiert sein kann, ein Cycloalkyl mit 3 bis 6 Kohlenstoffatomen, ein Alkoxy mit 1 bis 3 Kohlenstoffatomen, Cyan, ein Alkoxycarbonyl mit 2 bis 4 Kohlenstoffatomen oder ein Alkylthio mit 1 bis 3 Kohlenstoffatomen darstellen, wobei $R_4$ und $R_5$ nicht gleichzeitig Wasserstoff sind;

X Vinylen oder Azomethin ist;

A ein Alkylen mit 2 bis 4 Kohlenstoffatomen ist; und

B = $-N(R_6)_2$ oder

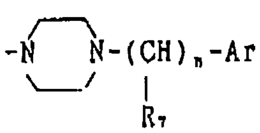

ist;
wobei

$R_6$ und $R_7$ unabhängig voneinander Wasserstoff, ein Alkyl mit 1 bis 6 Kohlenstoffatomen, das mit einem Cycloalkyl mit 3 bis 6 Kohlenstoffatomen substituiert sein kann, ein Cycloalkyl mit 3 bis 6 Kohlenstoffatomen, ein Aralkyl, ein Aryl oder Pyridyl sein kann;

Ar ein Aryl oder Pyridyl ist; und

n eine ganze Zahl von 0 bis 2 ist;

oder eines Säureadditionssalzes davon und eines pharmazeutisch annehmbaren Trägers oder Excipienten.

2. Verfahren gemäß Anspruch 1, wobei im Dihydropyridin-Derivat der Formel (I)

   $R_1$, $R_2$ und $R_3$ gleich oder verschieden sind und ein Alkyl darstellen;
   $R_4$ Wasserstoff ist;
   $R_5$ Nitro, ein halogeniertes Alkyl oder Cyan ist;
   $R_6$ ein Alkyl, ein Aralkyl oder ein Aryl ist;
   $R_7$ ein Aryl ist;
   Ar ein Aryl ist; und
   n = 1 ist.

3. Verfahren gemäß Anspruch 1, wobei das Dihydropyridin-Derivat aus 2-[p-(4-Benzhydrylpiperazino)phenyl]ethylmethyl-2,6-dimethyl-4-(3-nitrophenyl)-1,4-dihydropyridin-3,5-dicarboxylat und 2-[p-(4-Benzhydrylpiperazino)phenyl]ethylmethyl-2,6-dimethyl-4-(4-cyan-2-pyridyl)-1,4-dihydropyridin-3,5-dicarboxylat und ihren Säureadditionssalzen ausgewählt ist.

4. Verfahren gemäß Anspruch 1, wobei das Dihydropyridin-Derivat aus 2-[p-(4-Benzhydrylpiperazino)phenyl]ethylmethyl-2,6-dimethyl-4-(3-nitrophenyl)-1,4-dihydropyridin-3,5-dicarboxylat und 2-[p-(4-Benzhydrylpiperazino)phenyl]ethylmethyl-2,6-dimethyl-4-(4-cyan-2-pyrldyl)-1,4-dihydropyridin-3,5-dicarboxylat und ihren Hydrochloriden ausgewählt ist.

5. Verfahren gemäß Anspruch 1, wobei das Medikament für die Behandlung von Herzversagen geeignet ist.

6. Verfahren gemäß Anspruch 1 oder 2, wobei X im Dihydropyridin-Derivat der Formel (I) Vinylen ist.

7. Verfahren gemäß Anspruch 6, wobei es sich bei dem Dihydropyridin-Derivat um 2-[p-(4-Benzhydrylpiperazino)phenyl]ethylmethyl-2,6-dimethyl-4-(3-nitrophenyl)-1,4-dihydropyridin-3,5-dicarboxylat oder ein Säureadditionssalz davon handelt.

8. Verfahren gemäß Anspruch 6, wobei es sich bei dem Dihydropyridin-Derivat um 2-[p-(4-Benzhydrylpiperazino)phenyl]ethylmethyl-2,6-dimethyl-4-(3-nitrophenyl)-1,4-dihydropyridin-3,5-dicarboxylat oder ein Hydrochlorid davon handelt.

9. Verfahren gemäß Anspruch 6, wobei das Medikament für die Behandlung von Herzversagen geeignet ist.

**Revendications**

**Revendications pour les Etats contractants suivants : BE, CH, LI, DE, DK, FR, GB, IT, NL, SE**

1. Utilisation d'un dérivé de dihydropyridine de formule (I)

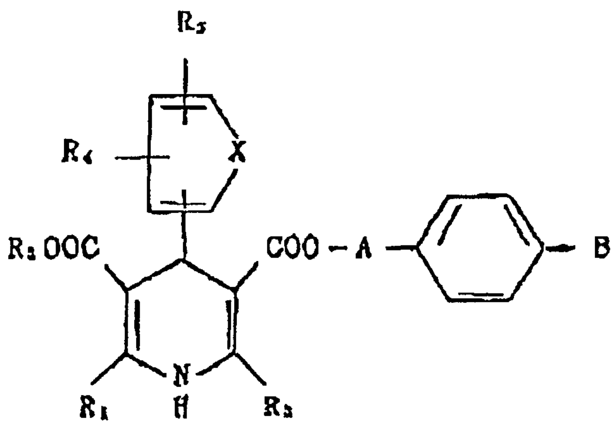

(I)

dans laquelle :

R1, R2 et R3 sont identiques ou différents et sont un alkyle en C1 à C6 qui peut être substitué par un cycloalkyle en C3 à C6, un cycloalkyle en C3 à C6 ou un alkoxyalkyle en C3 à C7;
R4 et R5 sont identiques ou différents et sont un hydrogène, un halogène, un nitro, un alkyle halogéné en C1 à C6, un alkylsulfonyle en C1 à C6, un alkoxy halogéné en C1 à C6, un alkylsulfinyle en C1 à C6, un alkylo en C1 à C6 qui peut être substitué par un cycloalkyle en C3 à C6, un cycloalkyle en C3 à C6, un alkoxy en C1 à C3, un cyano, un alkoxycarbonyle en C2 à C4 ou un alkylthio en C1 à C3, dans lesquels R4 et R5 ne sont pas en même temps un hydrogène :

X est un vinylène ou un azométhine;
A est un alkylène en C2 à C4; et
B est $-N(R_6)_2$ ou

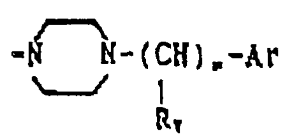

dans lesquelles
R6 et R7 sont indépendamment un hydrogène, un alkyle en C1 à C6 qui peut être substitué par un cycloalkyle en C3 à C6, un cycloalkyle en C3 à C6, un aralkyle, un aryle ou un pyridyle,
Ar est un aryle ou un pyridyle et
n est un nombre entier compris entre 0 et 2;

ou un sel d'addition acide de celle-ci et un support ou un excipient pharmaceutiquement acceptable, pour la production d'un médicament cardiotonique pour augmenter le débit cardiaque et la valeur différentielle maximum du pouls ventriculaire gauche.

2. Utilisation telle que revendiquée dans la revendication 1, de telle sorte que dans le dérivé de dihydropyridine de formule (I)

R1, R2 et R3 sont identiques ou différents et sont un alkyle;
R4 est un hydrogène;
R5 est un nitro, un alkyle halogéné ou un cyano;
R6 est un alkyle, un aralkyle ou un aryle;
R7 est un aryle;
Ar est un aryle; et
n vaut 1.

3. Utilisation telle que revendiquée dans la revendication 1, dans laquelle le dérivé de dihydropyridine est sélectionné

parmi le 2-[p-(4-benzhydrylpipérazino)phényl]éthyl méthyl 2,6-diméthyl-4-(3-nitrophényl)-1,4-dihydropyridine-3,5-dicarboxylate et le 2-[p-(4-benzhydrylpipérazino)phényl]-éthyl méthyl 2,6-diméthyl-4-(4-cyano-2-pyridyl-1,4-dihydropyridine-3,5-dicarboxylate et leurs sels d'addition acides.

**4.** Utilisation telle que revendiquée dans la revendication 1, dans laquelle le dérivé de dihydropyridine est sélectionné parmi le 2-[p-(4-benzhydrylpipérazino)phényl]éthyl méthyl 2,6-diméthyl-4-(3-nitrophényl)-1,4-dihydropyridine-3,5-dicarboxylate et le 2-[p-(4-benzhydrylpipérazino)phényl]-éthyl méthyl 2,6-diméthyl-4-(4-cyano-2-pyridyl)-1,4-dihydropyridine-3,5-dicarboxylate et leurs chlorhydrures.

**5.** Utilisation telle que revendiquée dans la revendication 1, dans laquelle le médicament est adapté pour le traitement de l'insuffisance cardiaque.

**6.** Utilisation telle que revendiquée dans la revendication 1 ou 2, moyennant quoi dans le dérivé de dihydropyridine de formule (I) X est un vinylène.

**7.** Utilisation telle que revendiquée dans la revendication 6, dans laquelle dans le dérivé de dihydropyridine est le 2-[p-(4-benzhydrylpipérazino) phényl] éthyl méthyl 2,6-diméthyl-4-(3-nitrophényl)-1, 4-dihydropyridine-3,5-dicarboxylate ou un sel d'addition acide de celui-ci.

**8.** Utilisation telle que revendiquée dans la revendication 6, dans laquelle dans le dérivé de dihydropyridine est le 2-[p-(4-benzhydrylpipérazino) phényl]éthyl méthyl 2,6-diméthyl-4-(3-nitrophényl)-1,4-dihydropyridine-3,5-dicarboxylate ou un chlorhydrure de celui-ci.

**9.** Utilisation telle que revendiquée dans la revendication 6, dans laquelle le médicament est adapté pour le traitement de l'insuffisance cardiaque.

**Revendications pour l'Etat contractant suivant : ES**

**1.** Procédé de fabrication d'un médicament cardiotonique destiné à augmenter le débit cardiaque et la valeur différentielle maximum du pouls ventriculaire gauche **caractérisé par** l'utilisation d'un dérivé de dihydropyridine de formule

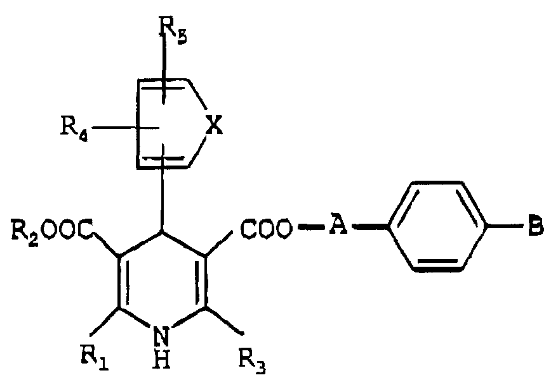

(I)

dans laquelle ;

$R_1$, $R_2$ et $R_3$ sont identiques ou différents et sont un alkyle ayant de 1 à 6 atomes de carbone qui peut être substitué par un cycloalkyle ayant de 3 à 6 atomes de carbone, un cycloalkyle ayant de 3 à 6 atomes de carbone ou un alcoxyalkyle ayant de 3 à 7 atomes de carbone ;
$R_4$ et $R_5$ sont identiques ou différents et sont un hydrogène, un halogène, un nitro, un alkyle halogéné ayant de 1 à 6 atomes de carbone, un alkylsulfonyle ayant de 1 à 6 atomes de carbone, un alcoxy halogéné ayant de 1 à 6 atomes de carbone, un alkylsulfinyle ayant de 1 à 6 atomes de carbone, un alkyle ayant de 1 à 6 atomes de carbone qui peut être substitué par un cycloalkyle ayant de 3 à 6 atomes de carbone, un cycloalkyle ayant de 3 à 6 atomes de carbone, un alcoxy ayant de 1 à 3 atomes de carbone, un cyano, un alcoxy-carbonyle ayant de 2 à 4 atomes de carbone ou un alkylthio ayant de 1 à 3 atomes de carbone, où $R_4$ et $R_5$ ne sont pas en même temps un hydrogène ;

X est un vinylène ou une azométhine ;
A est un alkylène ayant de 2 à 4 atomes de carbone ; et
B est $-N(R_6)_2$ ou

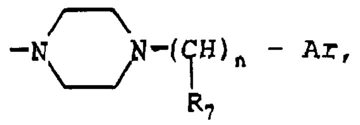

où
$R_6$ et $R_7$ sont indépendamment un hydrogène, un alkyle ayant de 1 à 6 atomes de carbone qui peut être substitué par un cycloalkyle ayant de 3 à 6 atomes de carbone, un cycloalkyle ayant de 3 à 6 atomes de carbone, un aralkyle, un aryle ou un pyridyle,
Ar est un aryle ou un pyridyle et
n est un nombre entier compris entre 0 et 2 ;

ou un sel d'addition d'acide de celui-ci et un véhicule ou excipient pharmaceutiquement acceptable.

**2.** Procédé selon la revendication 1 dans lequel, dans le dérivé de dihydropyridine de formule (I)

$R_1$, $R_2$ et $R_3$ sont identiques ou différents et sont un alkyle ;
$R_4$ est hydrogène ;
$R_5$ est un nitro, un alkyle halogéné ou un cyano ;
$R_6$ est un alkyle, un aralkyle ou un aryle ;
$R_7$ est un aryle ;
Ar est un aryle ; et
n vaut 1.

**3.** Procédé selon la revendication 1, dans lequel le dérivé de dihydropyridine est choisi parmi le 2,6-diméthyl-4-(3-nitrophényl)-1,4-dihydropyridine-3,5-dicarboxylate de 2-[p-(4-benzhydrylpipérazino)phényl]-éthylméthyle et le 2,6-diméthyl-4-(4-cyano-2-pyridyl)-1,4-dihydropyridine-3,5-dicarboxylate de 2-[p-(4-benzhydcylpipérazino)phényl]éthylméthyle et leurs sels d'addition d'acides.

**4.** Procédé selon la revendication 1, dans lequel le dérivé de dihydropyridine est choisi parmi le 2,6-diméthyl-4-(3-nitrophényl)-1,4-dihydropyridine-3,5-dicarboxylate de 2-[p-(4-benzhydrylpipérazino)phényl]-éthylméthyle et le 2,6-diméthyl-4-(4-cyano-2-pyridyl)-1,4-dihydropyridine-3,5-dicarboxylate de 2-[p-(4-benzhydrylpipérazino)phényl]éthylmethyle et leurs chlorhydrates.

**5.** Procédé selon la revendication 1, dans lequel le médicament est utile pour le traitement de l'insuffisance cardiaque.

**6.** Procédé selon la revendication 1 ou 2 dans lequel, dans le dérivé de dihydropyridine de formule (I) X est un vinylène.

**7.** Procédé selon la revendication 6, dans lequel le dérivé de dihydropyridine est le 2,6-diméthyl-4-(3-nitrophényl)-1,4-dihydropyridine-3,5-dicarboxylate de 2-[p-(4-benzhydrylpipérazino)phényl]éthylméthyle ou un sel d'addition d'acide de celui-ci.

**8.** Procédé selon la revendication 6, dans lequel le dérivé de dihydropyridine est le 2,6-diméthyl-4-(3-nitrophényl)-1,4-dihydropyridine-3,5-dicarboxylate de 2-[p-(4-benzhydryl-pipérazino)phényl]éthylméthyle ou un chlorhydrate de celui-ci.

**9.** Procédé selon la revendication 6, dans lequel le médicament est utile pour le traitement de l'insuffisance cardiaque.